# NOUVEAU FASCICULE DE BREVET EUROPEEN

(11) **EP 0 874 686 B2**
(45) Date de publication et mention de la décision concernant l'opposition: **14.09.2005**
(45) Mention de la délivrance du brevet: 08.05.2002
(21) Numéro de dépôt: 96931116.6
(22) Date de dépôt: 12.09.1996
(51) Int. Cl.: C07C 209/48

(54) **Procédé d'hydrogénation de nitriles**
Verfahren zur Hydrierung von Nitrilen
Process for hydrogenating nitriles

(30) Priorité: 15.09.1995 FR 9511064
(43) Date de publication de la demande: 04.11.1998
(73) Titulaire: RHODIA POLYAMIDE INTERMEDIATES, 69192 Saint-Fons (FR)
(72) Inventeur: CORDIER, Georges, F-69340 Francheville (FR); POPA, Jean-Michel, Guanabara 13023-010 Campinas, SP (BR)
(74) Mandataire: Esson, Jean-Pierre
(86) Numéro de dépôt international: PCT/FR1996/001406
(87) Numéro de publication internationale: WO 1997/010052

(56) Documents cités:
- EP-A- 0 566 197
- EP-A- 0 742 045
- WO-A-94/14700
- WO-A-96/20166
- FR-A- 2 091 785
- GB-A- 1 336 865
- US-A- 4 598 058
- US-A- 5 232 887

## Description

La présente invention concerne des nouveaux composés métalliques, un procédé de préparation de ces composés métalliques et leur utilisation comme catalyseurs.

Parmi les nombreux composés métalliques, utilisés notamment comme catalyseurs dans diverses réactions chimiques, les catalyseurs d'hydrogénation à base de nickel ou de cobalt représentent une classe importante. Ce sont en particulier le nickel de Raney et le cobalt de Raney qui sont largement utilisés dans l'industrie pour de nombreuse réactions d'hydrogénation.

Ce type de catalyseurs, très efficaces et à domaines d'application très larges, présente néanmoins certains inconvénients et certaines limites d'utilisation.

Tout d'abord leur préparation se fait par attaque à l'aide d'une base forte d'un alliage comportant le nickel ou le cobalt et une forte proportion d'aluminium.

Une telle préparation implique donc nécessairement la génération de grosses quantités d'effluents aqueux basiques contenant des aluminates, effluents qu'il est indispensable de traiter.

Une autre limite à l'utilisation des nickel et cobalt de Raney tient à ce que leur forme réduite est pyrophorique ; elle doit être manipulée avec précautions et ne peut être mise en oeuvre que protégée, soit sous forme de suspension dans un liquide, soit sous forme enrobée dans un solide protecteur. Cela rend difficile une utilisation en lit fixe à l'échelle industrielle.

Enfin, on observe une désactivation progressive des nickel ou cobalt de Raney, tandis que l'on ne connaît pas de mode de régénération efficace. Une des hypothèses qui semble admise pour expliquer cette désactivation, notamment en milieu contenant de l'eau et plus spécifiquement en milieu basique, est l'oxydation progressive de l'aluminium résiduel en aluminate, qui recouvre la surface active du nickel ou du cobalt.

Une autre forme de catalyseur métallique a été mise en oeuvre, afin de pallier certains inconvénients précités des catalyseurs de Raney : les métaux sont déposés sur un support. Ainsi le brevet EP-A-0 566 197 décrit des catalyseurs d'hydrogénation à base de nickel et/ou de cobalt déposés sur un support solide, tel qu'un silicate d'aluminium, une alumine ou une silice, de préférence avec un cocatalyseur tel qu'un sel ou un oxyde de métal alcalin ou alcalino-terreux non acide.

Dans ce type de catalyseur l'homogénéité de la répartition du métal actif dans le support solide n'est pas toujours très bonne. En outre, la présence d'un support peut dans certains cas limiter l'activité du catalyseur et de toute façon dilue la phase métallique active.

Ainsi dans le brevet EP-A-0 566 197 les exemples montrent que la teneur en métal actif des catalyseurs supportés ne dépasse pas 20 % et est souvent de 5 %. Enfin, le mode de dépôt du métal actif par imprégnation du support, peut entraîner une dissolution partielle du métal actif dans le milieu réactionnel, lors de la mise en oeuvre du catalyseur.

Il a également été proposé dans la demande de brevet WO962016 des catalyseurs d'hydrogénation à base de métaux choisis parmi le nickel, le cobalt, le fer, le ruthénium ou le rhodium et un promoteur métallique. Le catalyseur est obtenu par coprécipitation des ces éléments métalliques en présence ou non d'un support.

De même, le brevet EP742045, représentant un droit antérieur, décrit un catalyseur à base de cobalt et de promoteurs comprenant du phosphore, du manganese obtenu par coprécipitation de ces composés et calcination de la poudre obtenue. Ce catalyseur est notamment utilisé pour l'hydrogénation de nitriles en amines.

Ces catalyseurs présentent une dispersion du promoteur insuffisante pour obtenir un effet catalytique maximal.

La présente invention a pour objet un procédé d'hydrogénation de nitrile, caractérisé en ce que l'on opère en présence d'un catalyseur choisi parmi les composés métalliques comportant un ou plusieurs métaux divalents choisis parmi le nickel et le cobalt, au moins en partie à l'état réduit, texturés de manière homogène par une phase comprenant un ou plusieurs métaux dopants choisis parmi le chrome, le molybdène, le fer, le manganèse, le titane, le vanadium, le gallium, l'indium, le bismuth, l'yttrium, le cérium, le lanthane et les autres lanthanides trivalents, sous forme d'oxydes, le rapport molaire métal dopant/métal divalent étant compris entre 0,01 et 0,50, ledit catalyseur étant susceptible d'être préparé selon un procédé comprenant les étapes suivantes:
- préparation de composés comprenant les éléments métalliques contenus dans le catalyseur et ayant une structure appartenant à la famille des doubles hydroxydes lamellaires (DHL) de type hydrotalcite,
- calcination desdits composés de type hydrotalcite, et
- réduction d'au moins une partie des métaux divalents à l'état d'oxydation zéro.

Les catalyseurs selon l'invention possèdent, un dans la catalyse de la réaction d'hydrogénation, une efficacité du même ordre que celle obtenue avec les nickel ou cobalt de Raney, tout en ne présentant pas les inconvénients indiqués précédemment, notamment en ce qui concerne leur désactivation et leur régénération.

En outre, ne comportant pas de support, ils ne sont constitués pratiquement que de composés actifs.

Ces composés métalliques sont plus précisément des composés comportant un ou plusieurs métaux divalents au moins en partie à l'état réduit, texturés par une phase comprenant un ou plusieurs métaux dopants choisis parmi le chrome, le molybdène, le fer, le manganèse, le titane, le vanadium, le gallium, l'indium, le bismuth, l'yttrium, le cérium, le lanthane et les autres lanthanides trivalents, sous forme d'oxydes.

Les métaux présents sous forme d'oxydes sont appelés métaux dopants dans le présent texte, car ils sont nécessaires aux métaux divalents pour exercer de manière optimale leur activité catalytique, notamment parce qu'ils confèrent à l'ensemble une surface spécifique suffisante.

Les métaux divalents sont de préférence le nickel ou le cobalt.

Ils sont en général pour au moins 20 % de leurs atomes à l'état réduit, c'est-à-dire au degré d'oxydation 0. De préférence, au moins 50 % des atomes de nickel ou de cobalt sont à l'état réduit.

Les métaux divalents se présentent sous forme de particules ayant des dimensions situées généralement entre 1 et 20 nanomètres. Plus spécifiquement, la taille des particules de métal divalent est de 3 à 5 nanomètres. Ces tailles de particules sont mesurées par diffraction de rayons X.

Les particules de métal divalent sont texturées de manière homogène avec les particules d'au moins un oxyde de métal dopant choisi parmi les métaux indiqués précédemment. Les oxydes de métal dopant présentent des tailles de particules ayant le même ordre de grandeur que les particules de métal divalent.

La surface spécifique des composés métalliques de l'invention est généralement comprise entre 20 m²/g et 150 m²/g,

Le rapport molaire métal dopant/métal divalent dans les composés métalliques de l'invention est généralement compris entre 0,01 et 0,50.

De préférence ce rapport molaire est de 0,05 à 0,30.

Une partie des métaux divalents, nickel ou cobalt, peut être substituée par un ou plusieurs autres métaux, tels que notamment le zinc, le cuivre, l'argent, l'or, le ruthénium, le platine ou le palladium. Ces métaux peuvent représenter en moles par mole de nickel et/ou cobalt de 0 % à 50 %.

Pour le rapport métal dopant/métal divalent défini précédemment, ces autres métaux éventuellement présents sont considérés comme faisant partie de l'ensemble métal divalent.

De même les oxydes de métal dopant peuvent être partiellement remplacés par de l'oxyde d'aluminium. Cet oxyde d'aluminium peut représenter de 0 % à 50 % en moles d'aluminium par mole de la totalité des métaux dopants présents.

Les métaux dopants présents sous forme d'oxydes dans les composés métalliques de l'invention sont généralement au degré d'oxydation 3, mais peuvent pour certains d'entre-eux être au moins partiellement aux degrés d'oxydation 4 ou 5.

Un autre objet de l'invention concerne les précurseurs des composés métalliques décrits précédemment.

Ces précurseurs se présentent comme des composés métalliques comportant un ou plusieurs métaux divalents à l'état d'oxydes, texturés par une phase comprenant un ou plusieurs métaux dopants choisis parmi le chrome, le molybdène, le fer, le manganèse, le titane, le vanadium, le gallium, l'indium, le bismuth, l'yttrium, le cérium, le lanthane et les autres lanthanides trivalents, sous forme d'oxydes.

Comme indiqué précédemment pour les composés métalliques dans lesquels le ou les métaux divalents sont au moins en partie à l'état réduit, de 0 % à 50 % en moles de ces oxydes de métal dopant peuvent être remplacés par de l'oxyde d'aluminium et de 0 % à 50 % en moles du ou des oxydes des métaux divalents peuvent être remplacés par des oxydes de zinc, de cuivre, d'argent, d'or, de ruthénium, de platine et/ou de palladium.

Les oxydes des métaux divalents de ces précurseurs, ainsi que les oxydes de la plupart des métaux pouvant remplacer une partie des métaux divalents, sont facilement réductibles. Les précurseurs permettent d'obtenir par réduction à température relativement modérée les composés métalliques de l'invention.

Cette réduction peut s'effectuer par chauffage sous hydrogène. La température de réduction est de préférence comprise entre 200°C et 500°C.

L'hydrogène peut être mis en oeuvre sous pression ou par balayage.

La durée de la réduction peut varier très largement. A titre indicatif, elle se situe généralement entre quelques minutes et 24 heures, le plus souvent entre 1 et 10 heures.

Les oxydes des métaux dopants, et notamment l'oxyde d'aluminium éventuellement présent, ne sont pas réduits dans les conditions de réduction des métaux divalents indiqués précédemment. Les composés métalliques de l'invention ne comportent donc pas d'aluminium au degré d'oxydation 0, à la différence des nickel ou cobalt de Raney.

Un procédé de synthèse des précurseurs des composés métalliques de l'invention consiste à préparer des composés ayant une structure hydrotalcite appartenant à la famille des doubles hydroxydes lamellaires (DHL) de formule (I) :

[M(II)₁₋ₓ M(III)ₓ (OH)₂]^{x+} (A_{x/n})ⁿ⁻, mH₂O (I)

dans laquelle :
- A représente un anion minéral tel que carbonate, sulfate, nitrate, lodate, halogénure, vanadate, chromate, molybdate, aluminate, stannate, zincate, permanganate, cuprate, gallate, un anion hétéropolyacide, un anion carboxylate, ou un mélange de plusieurs de ces anions,
- M(II) est un métal divalent,
- M(III) est un métal dopant au degré d'oxydation 3,
- x représente un nombre de 0,01 à 0,33,
- n représente la valence de l'anion A,
- m représente un nombre variable de molécules dépendant du mode de préparation et des conditions de séchage,
puis à calciner lesdits composés de type hydrotalcite.

Las hydrotalcites (ou DHL) sont appelées ainsi par extension du nom du composé naturel Mg₆ Al₂ (OH)₁₆ CO₃, 4 H₂O.

Dans le cas des DHL conduisant aux précurseurs des composés métalliques de l'invention, M(II) représente au moins en partie Ni ou Co et M(III) représente au moins un métal dopant au degré d'oxydation 3, choisi parmi le chrome, le molybdène, le fer, le manganèse, le titane, le vanadium, le gallium, l'indium, le bismuth, l'yttrium, le cérium, le lanthane et les autres lanthanides trivalents.

Les hydrotalcites de formule (I) préférées sont celles pour lesquelles A représente un anion carbonate, nitrate, vanadate, chromate, molybdate, aluminate, stannate, zincate, permanganate, cuprate, gallate, carboxylate ou un mélange de plusieurs de ces anions et x représente un nombre de 0,048 à 0,23.

Les hydrotalcites de formule (I) sont préparées par précipitation, lors du mélange de solutions aqueuses de composés minéraux des métaux divalents et dopants entrant dans la composition desdites hydrotalcites, et d'un carbonate, notamment de métal alcalin.

Les composés qui peuvent être utilisés sont les sels solubles dans l'eau des différents métaux M(II) et M(III).

A titre d'exemples non limitatifs de tels composés, on peut citer le nitrate de nickel,
le chlorure de nickel, le bromure de nickel, l'iodure de nickel, le sulfate de nickel,
le bromure de cobalt, le chlorure de cobalt, l'iodure de cobalt, le nitrate de cobalt,
le sulfate de cobalt, l'acétate de cuivre, le chlorure de cuivre, le nitrate de cuivre,
le sulfate de cuivre, le fluorure d'argent, le nitrate d'argent, l'acétate de zinc, le bromure de zinc, le chlorure de zinc,
le formiate de zinc, le nitrate de zinc, le sulfate de zinc,
le chlorure de chrome, le sulfate de chrome, le bromure de chrome, le bromure de fer,
le chlorure de fer, le formiate de fer, le nitrate de fer, l'oxalate de fer, le sulfate de fer,
le chlorure de titane, le bromure de titane, le bromure de gallium, le chlorure de gallium, le nitrate de gallium, le sulfate de gallium, le bromure d'indium, le chlorure d'indium,
le nitrate d'indium, le sulfate d'indium, le bromure de vanadium, l'iodure de vanadium,
le sulfate de vanadyle, l'acétate de cénum, le bromure de cérium, l'iodure de cérium,
le nitrate de cérium, l'acétate de lanthane, le bromure de lanthane, l'iodure de lanthane,
le nitrate de lanthane, le nitrate de bismuth, le nitrate de molybdène, le nitrate de manganèse.

La dissolution de ces composés et la précipitation de l'hydrotalcite de formule (I) sont effectuées à une température égale ou inférieure à 100°C.

Les hydrotalcites de formule (I) pour lesquelles A est un anion autre que l'anion carbonate peuvent être obtenues à partir de l'hydrotalcite-carbonate préparée précédemment, par échange des anions carbonates avec d'autres anions A en milieu aqueux.

Il est important de laver l'hydrotalcite pour éliminer la plus grande partie des cations et anions minéraux non précipités, provenant des composés mis en oeuvre.
Ces ions sont indésirables car ils sont susceptibles, au moins pour certains de favoriser un frittage des particules de métal divalent, d'où augmentation de la taille de celles-ci et diminution de la surface spécifique du précurseur et du composé métallique qui seront préparés à partir des hydrotalcites.

Les hydrotalcites ainsi obtenues sont ensuite séchées, puis calcinées pour former les précurseurs des composés métalliques de l'invention.

Cette calcination se fait généralement à une température de 250°C à 600°C, généralement sous ventilation d'air. La température de calcination sera adaptée dans la zone définie, à la nature des métaux dopants ainsi qu'au rapport métal dopant/métal divalent.

La durée de calcination est très variable. A titre indicatif, elle se situe le plus souvent entre quelques minutes et 24 heures.

Les composés métalliques définis précédemment peuvent être utilisés comme catalyseurs dans de nombreuses réactions. On peut d'une manière générale les mettre en oeuvre dans les réactions catalysées par les nickel ou cobalt de Raney.

Ce sont donc plus spécifiquement des catalyseurs d'hydrogénation. On peut ainsi les utiliser pour l'hydrogénation
- de diverses familles de composés azotés comme les nitriles, les imines, les oximes, les hétérocycles azotés, les composé azoïques, les composés nitrés,
- de composés carbonylés comme les sucres par exemple,
- de composés à insaturation carbone-carbone, comme par exemple les composés éthyléniques,
- de composés à cycle aromatique, comme par exemple les composés benzéniques ou naphtaléniques.

Parmi les substrats dont l'hydrogénation à l'aide d'hydrogène peut être catalysée par les composés métalliques de l'invention, les nitriles sont préférés.

L'invention concerne un procédé d'hydrogénation des mononitriles ou des dinitriles, aliphatiques, cycloaliphatiques, hétérocycliques ou aromatiques utilisant comme catalyseurs les composés métalliques décrits ci-dessus.

Les dinitriles représentent une famille particulièrement intéressante en raison des composés auxquels ils conduisent.

Ces dinitriles sont, plus particulièrement mais non limitativement, les substrats nitriles de formule (II) :

NC-R-CN (II)

dans laquelle R représente un groupement alkylène ou alcénylène, linéaire ou ramifié, ayant de 1 à 12 atomes de carbone, ou un groupement arylène ou aralkylène ou aralcénylène substitué ou non.

De préférence, on met en oeuvre des dinitriles de formule (II) dans laquelle R représente un radical alkylène, linéaire ou ramifié, ayant de de 1 à 6 atomes de carbone.

A titre d'exemples de tels dinitriles, on peut citer notamment l'adiponitrile, le méthylglutaronitrile, l'éthylsuccinonitrile, le diméthylsuccinonitrile, le malononitrile, le succinonitrile et le glutaronitrile et leurs mélanges, notamment les mélanges adiponitrile, méthylglutaronitrile, éthylsuccinonitrile qui proviennent d'un même procédé de synthèse de l'adiponitrile.

De façon privilégiée, la réaction d'hydrogénation des dinitriles est conduite en présence d'une base forte. Cette base forte est choisie de préférence parmi les composés suivants : LiOH, NaOH, KOH, RbOH, CsOH et leurs mélanges.

En pratique, on utilise le plus souvent NaOH et KOH, pour un bon compromis performance-prix, bien que RbOH et CsOH donnent des résultats encore meilleurs.

Le milieu réactionnel d'hydrogénation est de préférence liquide. Il contient au moins un solvant apte à solubiliser le substrat nitrile à hydrogéner, car cette réaction s'opère mieux lorsque ledit substrat se trouve en solution.

Suivant une modalité intéressante du procédé d'hydrogénation, on utilise un milieu réactionnel liquide au moins partiellement aqueux. L'eau est généralement présente dans une quantité inférieure ou égale à 50 %, avantageusement inférieure ou égale à 20 % en poids par rapport au milieu réactionnel total. Plus préférentiellement encore, la teneur en eau du milieu réactionnel est comprise entre 0,1 et 15 % en poids par rapport à l'ensemble des constituants dudit milieu.

En complément ou en substitution à l'eau, on peut prévoir au moins un autre solvant, du type alcool et/ou amide. Les alcools qui conviennent plus particulièrement sont par exemple le méthanol, l'éthanol, le propanol, l'isopropanol, le butanol, les glycols, tels que l'éthylène et/ou le propylène glycol, des polyols et/ou des mélanges desdits composés.

Dans le cas où le solvant est constitué par un amide, il peut s'agir par exemple de la N-méthylpyrrolidone ou du diméthylacétamide.

Lorsqu'il est employé avec l'eau, le solvant, de préférence alcoolique, représente de 1 % à 1000 % en poids par poids d'eau et de préférence de 2 % à 300 %.

Selon une autre caractéristique préférée de l'hydrogénation, on incorpore de la diamine, qui se forme dans la réaction, au sein du milieu réactionnel. Il s'agit par exemple d'hexaméthylènediamine, lorsque le substrat nitrile est l'adiponitrile.

La concentration de l'amine visée dans le milieu réactionnel est avantageusement comprise entre 50 % et 99 % en poids par rapport à la totalité du solvant inclus dans ledit milieu réactionnel et, plus préférentiellement encore, est comprise entre 60 % et 99 % en poids.

L'hydrogénation en phase liquide peut être conduite en discontinu (batch) sur du nitrile seul ou éventuellement additionné d'autres composés liquides, comme la diamine qui se forme dans la réaction et/ou le ou les solvants.

Elle peut également être conduite avec une introduction continue du substrat nitrile.

Lorsque l'on opère avec du nitrile et un ou plusieurs solvants et/ou de la diamine, l'introduction du substrat nitrile, par exemple l'adiponitrile, dans le milieu réactionnel se fait en respectant une concentration comprise entre 0,001 % et 30 % en poids par rapport au poids total (p/p) du milieu réactionnel liquide et de préférence entre 0,1 % et 20 % p/p.

La quantité de base dans le milieu réactionnel varie en fonction de la nature du milieu réactionnel.

Dès lors que le milieu réactionnel ne contient que de l'eau et de l'amine visée, à titre de milieu solvant liquide, la quantité de base est avantageusement supérieure ou égale à 0,1 mol/kg de catalyseur, de préférence comprise entre 0,1 et 2 mol/kg de catalyseur et plus préférentiellement encore entre 0,5 et 1,5 mol/kg de catalyseur.

Dans le cas où le milieu réactionnel comprend de l'eau et un alcool et/ou un amide, la quantité de base est supérieure ou égale à 0,05 mol/kg de catalyseur, est comprise de préférence entre 0,1 et 10,0 mol/kg et plus préférentiellement encore entre 1,0 et 8,0 mol/kg.

La quantité de catalyseur n'est pas critique, car selon le mode de mise en oeuvre du procédé, il peut être en quantité plusieurs fois plus importante que le substrat à hydrogéner, notamment lorsque celui-ci est introduit en continu dans le milieu réactionnel contenant le catalyseur. A titre indicatif, le catalyseur peut représenter de 0,1 % à 100 % en poids du poids du milieu réactionnel liquide et le plus souvent de 1 % à 50 %.

Une fois arrêtée la composition du milieu réactionnel et le choix du catalyseur, on procède à un mélange de ces deux éléments, puis on chauffe ce mélange à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur

Dans un mode de fonctionnement discontinu, elle peut varier de quelques minutes à plusieurs heures.

Dans un mode de fonctionnement continu, qui est parfaitement envisageable pour ce type de réaction, la durée n'est évidemment pas un paramètre figeable.

Il est à noter que l'homme du métier peut moduler la chronologie des étapes du procédé, selon les conditions opératoires. L'ordre donné ci-avant ne correspond qu'à une forme préférée, mais non limitative, du procédé d'hydrogénation.

Les autres conditions qui régissent l'hydrogénation (en mode continu ou discontinu), relèvent de dispositions techniques traditionnelles et connues en elles-mêmes.

Grâce à toutes les dispositions avantageuses évoquées ci-dessus, les composés métalliques permettent d'hydrogéner des substrats nitriles en amines, de façon sélective, rapide, commode et économique.

L'hydrogénation d'adiponitrile en hexaméthylènediamine est particulièrement importante, puisque ce dérivé hydrogéné est l'un des monomères de base de la fabrication du polyamide 6-6.

L'hydrogénation des dinitriles peut également donner accès à des aminonitriles. Ainsi, il est possible d'hydrogéner une seule des deux fonctions nitriles de l'adiponitrile pour obtenir l'aminocapronitrile. Ce dernier composé est aisément transformable par hydrolyse cyclisante en caprolactame, qui est le produit de départ d'une autre grande synthèse industrielle de polyamide, à savoir le polyamide-6.

L'invention est illustrée par les exemples suivants de préparation de composés métalliques de l'invention et d'application de ceux-ci dans l'hydrogénation d'adiponitrile en hexaméthylènediamine.

### EXEMPLES

### Exemples de préparation de composés métalliques selon l'invention

### Exemple 1

On prépare 200 ml d'une solution aqueuse A contenant 0,3 mol de Ni et de Cr, sous forme de leurs nitrates Ni(NO₃)₂, 6 H₂O et Cr(NO₃)₃, 8 H₂O, le rapport molaire Ni/Cr étant de 3.

On prépare 200 ml d'une solution aqueuse B contenant 0,4 mol de carbonate de sodium.

On coule régulièrement en quelques minutes la solution A dans la solution B, sous agitation, les deux solutions étant à une température de 80°C.

On observe la formation d'un précipité homogène. On maintient le mélange à 80°C sous agitation pendant environ 20 min.

On filtre le précipité et on le lave sur le filtre avec 1500 ml d'eau à 80°C.

Le précipité est alors séché dans une étuve ventilée, à 120°C pendant 12 h ; on obtient une poudre de couleur verte de formule : Ni₆Cr₂(OH)₁₆CO₃, 4H₂O.

Ce composé est ensuite calciné dans un four ventilé, pendant 3 h à 300°C. On élimine ainsi l'eau chimisorbée et CO₂. Le précurseur du composé métallique selon l'invention est constitué d'un oxyde mixte de Ni et de Cr très faiblement cristallisé.

Le précurseur est ensuite réduit par l'hydrogène, à 350°C pendant 29 h. On obtient un composé métallique selon l'invention constitué de Ni au degré d'oxydation 0 et d'oxyde de Cr (rapport molaire Ni/Cr = 3) ; ce composé se présente sous la forme d'une poudre noire ayant une surface spécifique de 70 m²/g: composé (a).

### Exemples 2 à 4

On répète l'exemple 1, en adaptant les quantités respectives de nitrate de Ni et de nitrate de Cr, de manière à avoir un rapport molaire Ni/Cr de 5 (exemple 2), 10 (exemple 3) et 20 (exemple 4).

Après les différentes phases de la synthèse décrite dans l'exemple 1, on obtient les trois composés métalliques selon l'invention suivants :
- composé (b) : poudre noire constituée de Ni et d'oxyde de Cr avec un rapport molaire Ni/Cr = 5, ayant une surface spécifique de 103 m²/g;
- composé (c) : poudre noire constituée de Ni et d'oxyde de Cr avec un rapport molaire Ni/Cr =10, ayant une surface spécifique de 80 m²/g;
- composé (d) : poudre noire constituée de Ni et d'oxyde de Cr avec un rapport molaire Ni/Cr = 20, ayant une surface spécifique de 30 m²/g.

### Exemples 5 à 10

En suivant le mode opératoire décrit dans l'exemple 1, on rajoute à la solution A la quantité de nitrate de cuivre Cu(NO₃)₂, 6H₂O ou de nitrate de zinc Zn(NO₃)₂, 6H₂O nécessaire pour avoir un rapport molaire Ni/Cu de 5, 10 ou 20 ou un rapport molaire Ni/Zn de 5, 10 ou 20. Le rapport molaire Ni+Cu/Cr ou Ni+Zn/Cr est de 5 pour tous les exemples.

Après les différentes phases de la synthèse décrite dans l'exemple 1, on obtient les six composés métalliques selon l'invention suivants :
- composé (e) : poudre noire constituée de Ni, de Cu et d'oxyde de Cr avec un rapport molaire Ni/Cu = 5 et un rapport molaire Ni+Cu/Cr = 5, ayant une surface spécifique de 85 m²/g ;
- composé (f) : poudre noire constituée de Ni, de Cu et d'oxyde de Cr avec un rapport molaire Ni/Cu = 10 et un rapport molaire Ni+Cu/Cr = 5, ayant une surface spécifique de 75 m²/g ;
- composé (g) : poudre noire constituée de Ni, de Cu et d'oxyde de Cr avec un rapport molaire Ni/Cu = 20 et un rapport molaire Ni+Cu/Cr = 5, ayant une surface spécifique de 70 m²/g ;
- composé (h) : poudre noire constituée de Ni, de Zn et d'oxyde de Cr avec un rapport molaire Ni/Zn = 5 et un rapport molaire Ni+Zn/Cr = 5, ayant une surface spécifique de 95 m²/g;
- composé (j): poudre noire constituée de Ni, de Zn et d'oxyde de Cr avec un rapport molaire Ni/Zn = 10 et un rapport molaire Ni+Zn/Cr = 5, ayant une surface spécifique de 87 m²/g;
- composé (k) : poudre noire constituée de Ni, de Zn et d'oxyde de Cr avec un rapport molaire Ni/Zn = 20 et un rapport molaire Ni+Zn/Cr = 5, ayant une surface spécifique de 82 m²/g.

### Exemples 11 et 12

En suivant le mode opératoire décrit dans l'exemple 1, on fait une solution A avec les quantités de nitrate de Ni(NO₃)₂, 6 H₂O et de nitrate de cérium Ce(NO₃)₃, 6H₂O ou de nitrate de Ni, de nitrate de chrome Cr(NO₃)₃, 8 H₂O et de nitrate de cérium, nécessaires pour avoir un rapport molaire Ni/Ce de 5 ou un rapport molaire Ni/Cr+Ce de 4.

Après les différentes phases de la synthèse décrite dans l'exemple 1, on obtient les deux composés métalliques selon l'invention suivants :
- composé (I) : poudre noire constituée de Ni et d'oxyde de Cr avec un rapport molaire Ni/Ce = 5, ayant une surface spécifique de 100 m²/g;
- composé (m) : poudre noire constituée de Ni, d'oxyde de Ce et d'oxyde de Cr avec un rapport molaire Ni/Ce+Cr = 4 et un rapport molaire Cr/Ce = 5, ayant une surface spécifique de 90 m²/g.

### Exemples d'utilisation de composés métalliques selon l'invention comme catalyseurs d'hydrogénation

### Exemples 13 à 22 et essai comparatif 1

On teste comme catalyseurs différents composés métalliques préparés précédemment.

Le test d'hydrogénation permet dans des conditions bien déterminées de comparer l'activité et la sélectivité de composés métalliques de compositions diverses.

L'appareillage utilisé consiste en un autoclave en acier inoxydable dans lequel est conduite la réaction, ledit autoclave est muni d'une ampoule de coulée en acier, résistant à la pression, d'une arrivée d'hydrogène ou de gaz inerte, d'un système autorégulé de chauffage , de moyens de mesure et de régulation de la pression et de la température, d'un barreau aimanté toumant à 1500 tours/minute et d'une sortie pour les gaz.

On charge dans l'autoclave le composé métallique selon l'invention (0,2 g de Ni) avec soit (1) 40 g d'éthanol et 1,6 g d'eau (exemples 13 à 16 et essai comparatif 1), soit (2) 0,3 ml d'éthanol, 38 g d'hexaméthylènediamine, 4 g d'eau distillée (exemples 17 à 22) et l'hydroxyde de métal alcalin (soude sauf mention différente) à raison de 2 mol/kg de Ni du composé métallique. On homogénéise le mélange et on le recouvre par de l'argon. On purge ensuite par de l'azote, puis par de l'hydrogène. Ensuite, on chauffe à la température (80°C) choisie et on établit la pression (25 bar) choisie.

On introduit 1 g d'adiponitrile (AdN) dans l'ampoule de coulée et on purge celle-ci trois fois à l'hydrogène. L'injection de l'AdN est effectuée en 1 h.

A titre de comparaison, un essai est effectué dans les mêmes conditions que les exemples 13 à 16 avec le milieu solvant (1), en mettant en oeuvre un nickel de Raney comportant 4,5 % de Cr et ayant une surface spécifique de 70 m² (rapport molaire Ni/Cr d'environ 20). Ce nickel de Raney comporte environ 7 % en poids d'AI métal par rapport au Ni.

En fin de réaction, on laisse en température et pression jusqu'à la fin de consommation d'hydrogène. L'hydrogénat est dosé par chromatographie en phase vapeur. On détermine ainsi les sélectivités (S) des différents sous-produits obtenus.

Les sous-produits dosés sont les suivants :
HMI : Hexaméthylèneimine
AMCPA : Aminométhylcyclopentylamine
NEtHMD : N-éthylhexaméthylènediamine
DCH : Diaminocyclohexane cis et trans
BHT : Bis(hexaméthylènetriamine).

La sélectivité en HMD en pourcentage est donnée par la relation : 100 - somme des sélectivités des sous-produits. En effet, l'HMD constituant la majeure partie du solvant réactionnel, elle ne peut pas être dosée directement de manière très précise.
Par contre il a été vérifié que les sous-produits sont globalement tous identifiés.

Les sélectivités en chacun des sous-produits sont représentées par le pourcentage molaire du sous-produit formé par rapport à l'ADN transformé. Dans tous les exemples et essais comparatifs effectués, le taux de transformation de l'ADN (ainsi que celui de l'aminocapronitrile intermédiaire) est de 100 %. Les sélectivités sont exprimées par commodité en µmol de sous-produit par mol d'AdN transformé.

Le tableau 1 ci-après indique la référence du composé métallique utilisé, la nature des métaux divalents et dopants qui le constituent, le rapport molaire de ces métaux, les sélectivités en HMD et en sous-produits déterminées.

### Exemples 23 et 24

On répète les exemples 17 à 22 d'hydrogénation de l'AdN, dans les mêmes conditions opératoires et avec le milieu solvant (2), en mettant en oeuvre comme catalyseurs les composés métalliques (I) et (m).

On obtient les résultats rassemblés dans le tableau 2 ci-après.

**Tableau 2**

| | **Exemple 23/composé (I)** | **Exemple 24/composé (m)** |
|---|---|---|
| S % HMD | 99,0 | 99,0 |
| S HMI | 900 | 550 |
| S AMCPA | 3000 | 300 |
| S NEtHMD | 1250 | 550 |
| S DCH | 400 | 500 |
| S BHT | 3400 | 7600 |

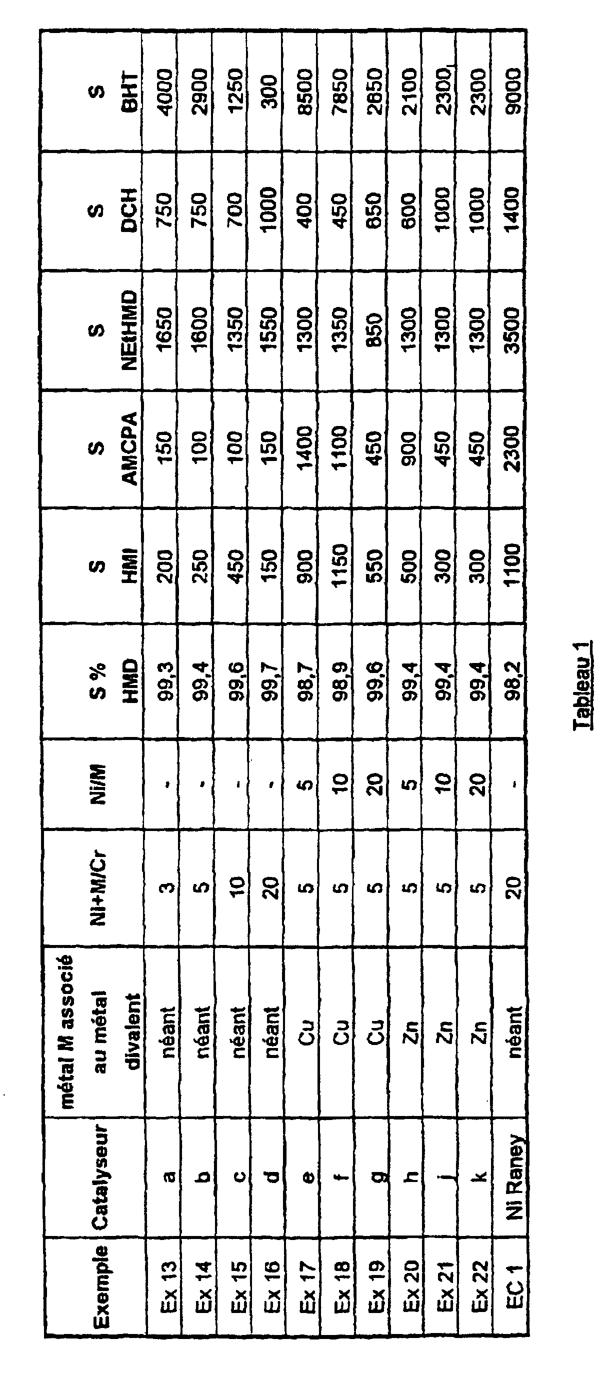

### Essai comparatif 2

### Préparation d'un composé métallique non compris dans l'invention : Ni/Al₂O₃

En suivant le mode opératoire décrit dans l'exemple 1, on fait une solution A avec les quantités de nitrate de Ni(NO₃)₂, 6 H₂O et de nitrate d'aluminium Al(NO₃)₃, 8H₂O nécessaires pour avoir un rapport molaire Ni/Al de 3.

Après les différentes phases de la synthèse décrite dans l'exemple 1, on obtient le composé métallique suivant:
- composé (p) : poudre noire constituée de Ni et d'oxyde d'AI avec un rapport molaire Ni/Al = 3, ayant une surface spécifique de 150 m²/g.

### Essai comparatif 3

### Utilisation du composé métallique (p) comme catalyseur d'hydrogénation.

On répète les exemples 13 à 16 d'hydrogénation de l'AdN, dans les mêmes conditions opératoires et avec le milieu solvant (1), en mettant en oeuvre comme catalyseur le composé métallique (p).

On obtient les résultats suivants:

| | |
|---|---|
| S % HMD | 81,3 |
| S HMI | 154000 |
| S AMCPA | 0 |
| S NEtHMD | 2500 |
| S DCH | 420 |
| S BHT | 15300 |
| S ACA* et CVA* | 15000 |

| | |
|---|---|
| (*) ACA = aminocaproamide ; CVA = cyanovaléramide | |

### Exemple 25 et essai comparatif 4

### Vieillissement du catalyseur dans une réaction d'hydrogénation.

Une série d'hydrogénations de l'ADN est effectuée dans les conditions opératoires décrites pour les exemples 13 à 22 avec le milieu (2), mais en injectant 10 g d'ADN en 1 h, d'une part avec le composé (d) selon l'invention (Ni/Cr = 20) et d'autre part avec le nickel de Raney utilisé dans l'essai comparatif 1 (Ni/Cr = 20).

On mesure le temps de finition Tf1 pour chaque hydrogénation, c'est-à-dire le temps pendant lequel l'absorption d'hydrogène continue, après la fin d'injection de l'ADN.

Avec chacun des deux catalyseurs, on réalise une deuxième injection de 10 g d'ADN en 1 h et on mesure le temps de finition Tf2.

On obtient les résultats suivants :
- exemple 25 : Tf1 = 15 min (S HMD = 99%)
   Tf2 =16 min
- essai comparatif 4 : Tf1 = 17 min (S HMD = 98,7%)
   Tf2 = 99 min.

On observe une rapide désactivation du catalyseur Raney alors que le catalyseur de l'invention conserve une activité constante.

## Revendications

1. Procédé d'hydrogénation de nitrile, **caractérisé en ce que** l'on opère en présence d'un catalyseur choisi parmi les composés métalliques comportant un ou plusieurs métaux divalents choisis parmi le nickel et le cobalt, au moins en partie à l'état réduit, texturés de manière homogène par une phase comprenant un ou plusieurs métaux dopants choisis parmi le chrome, le molybdène, le fer, le manganèse, le titane, le vanadium, le gallium, l'indium, le bismuth, l'yttrium, le cérium, le lanthane et les autres lanthanides trivalents, sous forme d'oxydes, le rapport molaire métal dopant/métal divalent étant compris entre 0,01 at 0,50, ledit catalyseur étant susceptible d'être préparé selon un procédé comprenant les étapes suivantes :
- préparation de composés comprenant les éléments métalliques contenus dans le catalyseur et ayant une structure hydrotalcite appartenant à la famille des doubles hydroxydes lamellaires (DHL) de formule générale (I) :
[M(II)₁₋ₓ M(III)ₓ (OH)2]^{x+} (Ax/n)ⁿ⁻, mH₂O (I)
dans laquelle :
- A représente un anion minéral tel que carbonate, sulfate, nitrate, iodate, halogénure, vanadate, chromate, molybdate, aluminate, stannate, zincate, permanganate, cuprate, gallate, un anion hétéropolyacide, un anion carboxylate, ou un mélange de plusieurs de ces anions,
- M(II) est un métal divalent,
- M(III) est un métal dopant au degré d'oxydation 3,
- x représente un nombre de 0,01 à 0,33,
- n représente la valence de l'anion A,
- m représente un nombre variable de molécules dépendant du mode de préparation et des conditions de séchage,
- calcination desdits composés, et
- réduction d'au moins une partie des métaux divalents à l'état d'oxydation zéro.

2. Procédé selon la revendication 1, **caractérisé en ce que** M(II) représente au moins en partie Ni ou Co, M(III) représente au moins un métal dopant au degré d'oxydation 3 choisi parmi le chrome, le molybdène, le fer, le manganèse, le titane, le vanadium, le gallium, l'indium, le bismuth, l'yttrium, le cérium, le lanthane et les autres lanthanides trivalents, A représente un anion carbonate, nitrate, vanadate, chromate, molybdate, aluminate, stannate, zincate, permanganate, cuprate, gallate, carboxylate ou un mélange de plusieurs de ces anions et x représente un nombre de 0,048 à 0,23.

3. Procédé selon l'une des revendications précédentes, **caractérisé en ce que** les métaux divalents sont pour au moins 20 % de leurs atomes à l'état réduit.

4. Procédé selon l'une des revendications précédentes, **caractérisé en ce que** les métaux divalents se présentent sous forme de particules ayant des dimensions situées entre 1 et 20 nanomètres et plus spécifiquement entre 3 et 5 nanomètres.

5. Procédé selon l'une des revendications précédentes, **caractérisé en ce que** 0 % à 50 %, en mole par mole, des métaux divalents, nickel ou cobalt, sont substitués par un ou plusieurs autres métaux choisis parmi le zinc, le cuivre, l'argent, l'or, le ruthénium, le platine et le palladium.

6. Procédé selon l'une des revendications précédentes, **caractérisé en ce que** de 0 % à 50 %, en mole par mole, des oxydes de métaux dopants sont substitués par de l'oxyde d'aluminium.

7. Procédé selon l'une des revendications précédentes, **caractérisé en ce que** les composés ayant une structure appartenant à la famille des doubles hydroxydes lamellaires (DHL) de type hydrotalcite sont préparés par précipitation, lors du mélange de solutions aqueuses de composés minéraux des métaux divalents et dopants entrant dans la composition desdites hydrotalcites, et d'un carbonate, notamment de métal alcalin, puis à calciner lesdits composés de type hydrotalcite à une température de 250°C à 600°C.

8. Procédé selon l'une des revendications précédentes, **caractérisé en ce que** le nitrile est choisi parmi les mononitriles ou les dinitriles aliphatiques, cycloaliphatiques, hétérocycliques ou aromatiques.

9. Procédé selon la revendication 8, **caractérisé en ce que** le nitrile est choisi parmi les substrats nitrile de formule (II):
NC-R-CN (II)
Dans laquelle R représente un groupement alkylène ou alcénylène, linéaire ou ramifié, ayant de 1 à 12 atomes de carbone, ou un groupement arylène ou aralkylène ou aralcénylène substitué ou non et de préférence un radical alkylène, linéaire ou ramifié, ayant de 1 à 6 atomes de carbone.

## Patentansprüche

1. Verfahren zur Hydrierung von Nitril, **dadurch gekennzeichnet, dass** man in Gegenwart eines Katalysators arbeitet, ausgewählt unter den Metallverbindungen, beinhaltend ein oder mehrere zweiwertige Metalle, ausgewählt unter Nickel und Kobalt, zumindest teilweise in reduzierter Form, homogen texturiert durch eine Phase, beinhaltend ein oder mehrere Dotierungsmetalle, ausgewählt unter Chrom, Molybdän, Eisen, Mangan, Titan, Vanadin, Gallium, Indium, Wismuth, Yttrium, Cer, Lanthan und den anderen dreiwertigen Lanthaniden, in Form von Oxiden, wobei das Molverhältnis Dotierungsmetall/zweiwertiges Metall zwischen 0,01 und 0,50 liegt, und wobei der Katalysator nach einem Verfahren hergestellt werden kann, das die folgenden Stufen beinhaltet:
- Herstellung von Verbindungen, umfassend die metallischen Elemente, die in dem Katalysator enthalten sind und eine Hydrotalcit-Struktur aufweisen, die der Familie der lamellaren Doppelhydroxide (DHL) der allgemeinen Formel (I)
[M(II)₁₋ₓM(III)ₓ(OH)₂]^{x+}(A_{x/n})ⁿ⁻, mH₂O (I)
angehört, worin:
- A ein mineralisches Anion wie Carbonat, Sulfat, Nitrat, Jodat, Halogenid, Vanadat, Chromat, Molybdat, Aluminat, Stannat, Zinkat, Permanganat, Cuprat, Gallat, ein heteropolysaures Anion, ein Carboxylat-Anion oder ein Gemisch von mehreren dieser Anionen bedeutet,
- M(II) ein zweiwertiges Metall ist,
- M(III) ein Dotierungsmetall mit dem Oxidationsgrad 3 ist,
- x eine Zahl von 0,01 bis 0,33 bedeutet,
- n die Wertigkeit des Anions A bedeutet,
- m eine variable Zahl der Moleküle wiedergibt, die von der Herstellungsart und den Trocknungsbedingungen abhängt,
- Kalzinierung besagter Verbindungen und
- Reduktion zumindest eines Teils der zweiwertigen Metalle in den Oxidationszustand Null.

2. Verfahren gemäß Anspruch 1, **dadurch gekennzeichnet, daß** M(II) zumindest teilweise Ni oder Co bedeutet, M(III) zumindest ein Dotierungsmetall mit dem Oxidationsgrad 3, ausgewählt unter Chrom, Molybdän, Eisen, Mangan, Titan, Vanadin, Gallium, Indium, Wismuth, Yttrium, Cer, Lanthan und den anderen dreiwertigen Lanthaniden, bedeutet, A ein Carbonat-, Nitrat-, Vanadat-, Chromat-, Molybdat-, Aluminat-, Stannat-, Zinkat-, Permanganat-, Cuprat-, Gallat-, Carboxylatanion oder ein Gemisch von mehreren dieser Anionen bedeutet, und x für eine Zahl von 0,048 bis 0,23 steht.

3. Verfahren gemäß einem der vorhergehenden Ansprüche, **dadurch gekennzeichnet, daß** die zweiwertigen Metalle zumindest 20% ihrer Atome in reduziertem Zustand vorliegen haben.

4. Verfahren gemäß einem der vorhergehenden Ansprüche, **dadurch gekennzeichnet, daß** die zweiwertigen Metalle in Form von Teilchen mit Dimensionen zwischen 1 und 20 Nanometem, und insbesondere zwischen 3 und 5 Nanometern, vorliegen.

5. Verfahren gemäß einem der vorhergehenden Ansprüche, **dadurch gekennzeichnet, daß** 0% bis 50% in Mol je Mol der zweiwertigen Metalle Nickel oder Kobalt durch ein oder mehrere weitere Metalle, ausgewählt unter Zink, Kupfer, Silber, Gold, Ruthenium, Platin und Palladium, substituiert sind.

6. Verfahren gemäß einem der vorhergehenden Ansprüche, **dadurch gekennzeichnet, daß** 0% bis 50% in Mol je Mol der Oxide der Dotierungsmetalle durch Aluminiumoxid substituiert sind.

7. Verfahren gemäß einem der vorhergehenden Ansprüche, **dadurch gekennzeichnet, daß** die Verbindungen mit einer Struktur, die der Familie der lamellaren Doppelhydroxide (DHL) vom Hydrotaleit-Typ angehört, hergestellt werden durch Ausfällen während des Mischens von wässrigen Lösungen der zweiwertigen mineralischen Verbindungen und Dotierungsmittel, die in die Zusammensetzung der Hydrotalcite eintreten, und eines Carbonats, insbesondere Alkalimetallcarbonats, und anschließendes Kalzinieren der Verbindungen vom Hydrotalcit-Typ bei einer Temperatur von 250 °C bis 600 °C.

8. Verfahren gemäß einem der vorhergehenden Ansprüche, **dadurch gekennzeichnet, daß** das Nitril unter den Mononitrilen oder den aliphatischen, cycloaliphatischen, heterocyclischen oder aromatischen Dinitrilen ausgewählt wird.

9. Verfahren gemäß Anspruch 8, **dadurch gekennzeichnet, daß** das Nitril unter den Nitrilsubstraten der Formel (II):
NC - R - CN (II)
ausgewählt wird, worin R eine lineare oder verzweigte Alkylen- oder Alkenylengruppe mit 1 bis 12 Kohlenstoffatomen oder eine substituierte oder unsubstituierte Arylen- oder Aralkylen- oder Aralkenylengruppe, vorzugsweise eine lineare oder verzweigte Alkylengruppe mit 1 bis 6 Kohlenstoffatomen, bedeutet.

## Claims

1. Process for the hydrogenation of a nitrile, **characterized in that** the hydrogenation is carried out in the presence of a catalyst chosen from metal compounds comprising one or more divalent metals chosen from nickel and cobalt, at least partially in the reduced state, homogeneously textured by a phase comprising one or more doping metals chosen from chromium, molybdenum, iron, manganese, titanium, vanadium, gallium, indium, bismuth, yttrium, cerium, lanthanum and the other trivalent lanthanides, in the form of oxides, the doping metal/divalent metal molar ratio being between 0.01 and 0.50, the said catalyst being capable of being prepared according to a process comprising the following stages:
- preparation of compounds comprising the metal elements present in the catalyst and having a hydrotalcite structure belonging to the family of the layered double hydroxides (LDHs) of general formula (I) :
[M(II)₁₋ₓM(III)ₓ(OH)₂]^{x+}(A_{x/n})ⁿ⁻-mH₂O (I)
in which:
- A represents an inorganic anion, such as carbonate, sulphate, nitrate, iodate, halide, vanadate, chromate, molybdate, aluminate, stannate, zincate, permanganate, cuprate or gallate, a heteropolyacid anion, a carboxylate anion or a mixture of several of these anions,
- M(II) is a divalent metal,
- M(III) is a doping metal with a degree of oxidation of 3,
- x represents a number from 0.01 to 0.33,
- n represents the valency of the anion A,
- m represents a variable number of molecules depending on the method of preparation and on the drying conditions,
- calcination of the said compounds, and
- reduction of at least a portion of the divalent metals to the zero oxidation state.

2. Process according to claim 1, **characterized in that** M(II) represents at least partially Ni or Co, M(III) represents at least one doping metal with a degree of oxidation of 3 chosen from chromium, molybdenum, iron, manganese, titanium, vanadium, gallium, indium, bismuth, yttrium, cerium, lanthanum and the other trivalent lanthanides, A represents a carbonate, nitrate, vanadate, chromate, molybdate, aluminate, stannate, zincate, permanganate, cuprate, gallate or carboxylate anion or a mixture of several of these anions, and x represents a number from 0.048 to 0.23.

3. Process according to either of the preceding claims, **characterized in that** the divalent metals are, with regard to at least 20% of their atoms, in the reduced state.

4. Process according to one of the preceding claims, **characterized in that** the divalent metals are provided in the form of particles having dimensions lying between 1 and 20 nanometres and more specifically between 3 and 5 nanometres.

5. Process according to one of the preceding claims, **characterized in that** 0% to 50%, as mol per mole, of the divalent metals, nickel or cobalt, are substituted by one or more other metals chosen from zinc, copper, silver, gold, ruthenium, platinum and palladium.

6. Process according to one of the preceding claims, **characterized in that** from 0% to 50%, as mol per mole, of the doping metal oxides are substituted by aluminium oxide.

7. Process according to one of the preceding claims, **characterized in that** the compounds having a structure belonging to the family of the layered double hydroxides (LDHs) of hydrotalcite type are prepared by precipitation during the mixing of aqueous solutions of divalent and doping metal mineral compounds participating in the composition of the said hydrotalcites and of a carbonate, in particular an alkali metal carbonate, and then by calcination of the said compounds of hydrotalcite type at a temperature of 250°C to 600°C.

8. Process according to one of the preceding claims, **characterized in that** the nitrile is chosen from aliphatic, cycloaliphatic, heterocyclic or aromatic mononitriles or dinitriles.

9. Process according to Claim 8, **characterized in that** the nitrile is chosen from the nitrile substrates of formula (II):
NC-R-CN (II)
in which R represents a linear or branched alkylene or alkenylene group having from 1 to 12 carbon atoms or an arylene or aralkylene or aralkenylene group which is substituted or unsubstituted and preferably a linear or branched alkylene radical having from 1 to 6 carbon atoms.
